# EUROPEAN PATENT SPECIFICATION

(11) **EP 1 696 741 B1**
(45) Date of publication and mention of the grant of the patent: **12.09.2007**
(21) Application number: 03818931.2
(22) Date of filing: 28.11.2003
(51) Int. Cl.: A23L 1/30, A61K 31/201

(54) **USE OF HYDROXYOLEIC ACID AND RELATED COMPOUNDS AS FUNCTIONAL FOOD ADDITIVES**
VERWENDUNG VON HYDROXYÖLSÄURE UND VERWANDTEN VERBINDUNGEN ALS FUNKTIONELLE NAHRUNGSMITTELZUSATZSTOFFE
UTILISATION D'ACIDE HYDROXYOLEIQUE ET DE COMPOSES APPARENTES COMME ADDITIFS ALIMENTAIRES DIRECTS

(30) Priority: 10.10.2003 ES 200302364
(43) Date of publication of application: 06.09.2006
(73) Proprietor: Universitat de les Illes Balears, Palma de Mallorca 07122 (ES)
(72) Inventor: ESCRIBA RUIZ, Pablo, Vicente, 07122 Palma de Mallorca (ES)
(74) Representative: González-Bueno, Pablo
(86) International application number: PCT/EP2003/013828
(87) International publication number: WO 2005/041691

(56) References cited:
- WO-A-02/00042
- WO-A-92/21335
- WO-A-03/030891
- US-A1- 2003 157 237
- MEIJER G W ET AL: "Effect of dietary elaidic versus vaccenic acid on blood and liver lipids in the hamster." July 2001 (2001-07), ATHEROSCLEROSIS. JUL 2001, VOL. 157, NR. 1, PAGE(S) 31 - 40 , XP002282516 ISSN: 0021-9150 page 31, paragraph 1 - page 32, paragraph 6; tables 1-4 page 39, paragraph 2

## Description

### FIELD OF THE INVENTION

The present invention relates to the use of hydroxyoleic (2-hydroxyoleic) acid and molecules of a similar structure as food additives with preventive properties with respect to the development of cardiovascular diseases and obesity, usually known as functional additives.

The present invention also relates to the use of 2-hydroxyoleic acid and similar compounds for the manufacture of food products.

### BACKGROUND TO THE INVENTION

Fatty acids are molecules of wide application in both foodstuffs and industry. Various dietary products contain this and other types of fats. Among them, mention may be made to oils (olive, soya, sunflower, evening primrose, sesame, etc.), (animal) fats/butters and (vegetable) margarines, whose composition consists of more than 95% fat. Many other food products contain vegetable and/or animal fats in greater or lesser quantities, which in some cases have preventive properties with respect to the development of various diseases, such as for example omega-3 fatty acids. Fatty acids are usually found in food and dietary products in two forms: free (usually at low percentages) or bound to other molecules such as phospholipids, cholesterol esters, triglycerides, etc. (main form of presentation of fatty acids). Whatever the form of presentation, in most food and dietary products, as in nature in general, fatty acids are not usually significantly modified. However, it has been demonstrated that there are large amounts of hydroxylated fatty acids (up to 60%) in the oils of some vegetable seeds (Moon et al., 2001, Plant Physiol. 1.27, 1635). Nevertheless, the abundance of hydroxylated fatty acids in nature varies significantly between different organisms and biological systems.

In this context, 2-hydroxyoleic acid, the synthesis of which has been described previously (Adam et al., 1998, Eur. J. Org. Chem. 9, 2013-2018), has been used industrially as an emulsifier for preparations of cosmetics. Hydroxylated fatty acids are known for their emulsifying ability, being used, as they are, for this purpose in food and in other applications.

Thus, for example, on the one hand, patent JP 10182338 relates to an oil-in-water emulsifying composition that exhibits low irritability and high compatibility with salts, which contains: [A] nonionic surfactants such as polyoxyethylene sorbitol monolaurate, polyoxyethylene sorbitol monooleate and polyoxyethylene sorbitol monostearate, [B] 2-hydroxy C10-C22 fatty acids such as 2-hydroxystearic acid, [C] oils and [D] water, in which the A/B ratio is between 1:0.01 and 1:2.

Along the same lines, as emulsifiers, patent JP 09110635 relates to compositions that can be used as pharmaceutical products, cosmetics and foodstuffs and contain: [A] esters of polyglyceryl fatty acid, [B] 2-hydroxy C10-C22 fatty acids, [C] oils and [D] water, where the weight ratio of A/C and B/C is 2.0 and 0.5 respectively, and have average particle sizes between 10 and 300 nm. These compositions show good stability even in acid conditions or at low viscosity or in the presence of elevated quantities of salts, and therefore are compatible with the skin.

Our earlier patent, WO 03/030891, disclosed the use of hydroxyoleic acid and similar compounds in the production of medicaments that can be used as anti-tumour agents, agents with hypotensive action and as agents capable of inducing weight loss. These therapeutic effects of the pharmaceutical compounds manufactured using hydroxyoleic acid and similar compounds, appeared to be caused by the regulation by these compounds of the membrane structure, thereby regulating the activity and/or location of G proteins and, consequently, the activity of the receptors connected thereto. The object of the present invention is now to claim the effect of prevention of cardiovascular diseases and obesity of hydroxyoleic acid and similar compounds, as nutritional dietary supplements.

### The hexagonal membrane structures.

The membrane lipids are able to arrange themselves in a greater number of secondary structures than the proteins and nucleic acids. The typical lipid bilayer of biological membranes is just one of these secondary configurations. Little is known about the abundance and roles of other secondary structures in living cells. One function of these structures was described in a previous work: to increase the binding affinity of G-proteins to membranes (Escribá PV, Ozaita A, Ribas C, Miralles A, Fodor E, Farkas and Garcia-Sevilla JA; 1997 Proceedings of the National Academy of Sciences of the USA 94, 11375-11380).

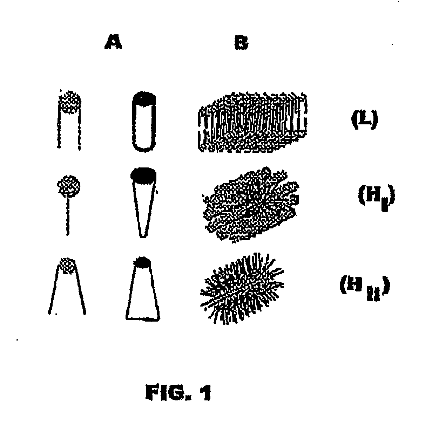

The concept of membrane structure goes far beyond that described in some of the patents of the state of the art (WO 87/04926 and WO 80/11286), in which only membrane fluidity is mentioned. In the invention, the concept is extended to a much wider field: the membrane structure. The molecules covered by our patent act on the transition or passage from lamellar to hexagonal structure (Fig. 1).

The receptors connected to G proteins are ubiquitous, constituting 80% of the membrane receptors which transmit signals initiated by neurotransmitters, hormones, neuromodulators, cytokines, growth factors, etc. They regulate several physiological processes, including blood pressure and body weight. The molecules described in this invention can therefore regulate said physiological processes.

Examining the prior art cited, in the state of the art there are as yet no other applications connected with 2-hydroxyoleic acid or similar compounds that would be of particular interest in the area of foodstuffs for the prevention of the development of cardiovascular diseases and obesity.

There are only descriptions of dietary products (GB 2140668, EP 0611568 and WO 02/0042) or extracts from cultures of M. cryophilus (WO 89/11286), which consist of complex mixtures of various compounds that include some similar to those described in the invention, such as fatty acids, in particular oleic and palmitoleic acids, for example, but without there being attributed to any of the components of the mixture envisaged in said patents a specific role in said therapeutic effect of preventing cardiovascular diseases.

The effect of the lipid composition of cells in the development of hypertension has been studied (Escribá et al., Hypertension 41, 176-182; 2003). In this regard, it has been observed that the differential composition of lipids which form the membrane is involved in the development of hypertension and the dietary control of the composition (and therefore of the structure) of the membrane is involved in the normalization thereof. This effect is regulated by the membrane structure's ability to control the location and activity of G proteins, as stated earlier.

We have knowledge of other patents in the prior art which describe the use, as drugs, of hydroxyoleic acid or similar compounds in indications related to those proposed herein.

Thus, WO 02/43662 dicloses a nutritional supplement based on linoleic acid, DHA, vitamins E, G, B6 and B12, and folic acid together with calcium, which can be used to reduce elevated cholesterol levels and high blood pressure. In addition, patent CN 1098920 discloses oral samara oil liquors (rich in linoleic acid and vitamin E) which can be used as hypotensive agents, hypocholesterolaemics and for the treatment of hepatitis, nephritis and other diseases. Linoleic acid is not the subject of this invention. US 5059622 and US 6140304 mention this same therapeutic indication, using eicosapentanoic and γ-linoleic acids. Hypertension linked to cerebral apoplexy has also been disclosed (GB 2140688), which can be treated using palmitoleic acid. In the prior art there are also diets including fatty acid di- and triglycerides as compounds which help to burn fat (WO 02/11552), in the case of omega-3 unsaturated fatty acids containing less than 20 carbon atoms, or which help weight loss (WO 01/91587), in the case of fatty acids having from 4 to 14 carbon atoms. Triglycerides are not the subject of this invention. WO 02/00042 discloses C12-24 fatty acids, particularly palmitoleic and oleic fatty acids, which induce a sensation of satiety. The weight control mechanism of the invention is not mediated by controlling the sensation of satiety.

As regards modifying the state of the membrane, this has already been disclosed (WO 89/11286 - palmitoleic; WO 87/0992.6 - C12-28 monounsaturated) but by means of mechanisms that relate to changes in fluidity, not structural transitions. Likewise, the use of nervonic acid is known as a nutritional supplement for new-born or premature babies and pregnant women (WO 96/05740), but for the prevention of diseases such as multiple sclerosis. Enteral preparations based on, inter alia, oleic acid, linoleic acid or eicosapentanoic acid have even been used for patients suffering from cancer (EP 0 611 568).

None of these indications is claimed in the present invention.

### Summary of the invention

The invention is best described by the claims.

The present invention has the aim of finding new applications of 2-hydroxyoleic acid and similar compounds that are not described in the state of the are and that are not obvious on the basis of that art.

The objective of the present invention is to show that 2-hydroxyoleic acid and its analogs have activity as functional food additives capable of preventing cardiovascular diseases by reducing blood pressure and causing weight loss. The addition of hydroxyoleic acid to food products for humans in amounts which in terms of consumption can be considered to be dietary (of the order of up to 0.5 g/kg of body weight per day) produces significant benefits in parameters related directly to cardiovascular diseases: blood pressure and body weight.

The applications described below for 2-hydroxyoleic acid and its analogs have not been cited by anyone previously and their use may prove beneficial for the prevention of certain diseases, through their inclusion in the diet. In particular, it has been found that 2-hydroxyoleic acid and its analogs display cardiovascular activity, inducing reductions in blood pressure and body weight.

In the present invention the new applications of 2-hydroxyoleic acid and its analogs are substantiated by using experimental cellular and animal models. These analysis models show, undoubtly, that 2-hydroxyoleic acid and its analogs are molecules that can be used for making drugs for human consumption, with the aim of preventing cardiovascular diseases.

The claimed use of the compounds of the invention should be understood as applying in the field of foodstuffs in its widest possible sense and they include, inter alia: food additives and/or ingredients, dietary products, acceptable food forms and foods in general.

### Detailed description of the invention

In the present invention, "2-hydroxyoleic acid" means, without distinction, α-hydroxyoleic acid, octadecenoic acid C18:1 cis Δ9 or cis-2-hydroxy-9-octadecenoic acid. "Their functional analogs" means those fatty acids that have the double bond shifted one or two positions from the central zone or that have the double bond shifted from one to five positions from the central zone and/or have from one to six carbon atoms (CH₂ groups) on each side of the double bond and/or that have a residue (R) in position 2 different from OH, with a small atomic mass and that is not H (Mw less than or equal to 200 Da). It does not matter whether the stereoisomer corresponding to the projection of the R group is R or S. In relation to the various molecules tested, it has been observed that those having the general formulas shown below display some similar effects to hydroxyoleic acid and can therefore be categorized as functional analogs thereof.

General formula I: COOH-CHR-(CH₂)ₘ-CH=CH-(CH₂)ₙ-CH₃ in which m and n have, independently, a value of 0-15 and R can be H, OH, NH₂, CH₃, or some other residue with molecular weight below 200 Da.

In the present invention "G-proteins" means proteins that are guanine nucleotide binding proteins, formed from three subunits (one alpha, one beta and one gamma) that transmit signals from receptors bound to G-proteins, to effectors (adenylyl cyclase, guanylyl cyclase, phospholipase C, ion channels, etc.).

In the present invention "membrane structure" means the secondary structure or arrangement of lipids in natural or synthetic membranes (liposomes).

In the present invention, "dietarily acceptable forms" means any form usually used in the sector, including, but not limited to: esters, especially ethyl esters owing to their solubilizing properties with respect to fatty acids, ethers, amides, salts, etc.

The term "functional food additive" should be understood as meaning additives which, when ingested, produce an effect of preventing the development of diseases which is greater than that inherent in the nutritional intake itself.

### Regulation of the activity of G-proteins controls blood pressure.

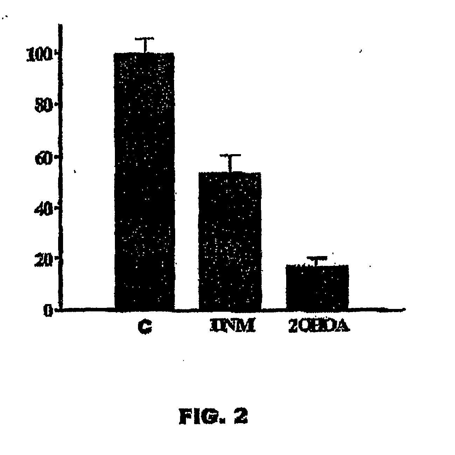

Hydroxyoleic acid and related compounds are capable of modulating the activity of G-proteins, measured by the binding of [³⁵S] GTPγS (Fig. 2).

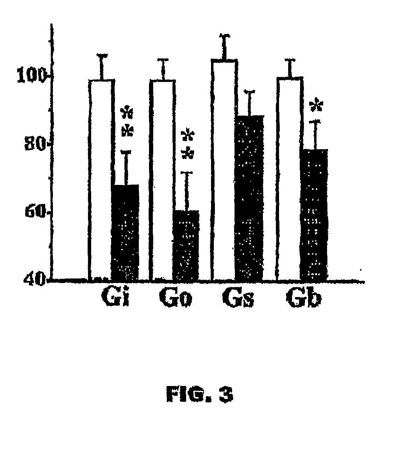

There is a relationship between the compounds of the invention and the activity of G-proteins and blood pressure. A result that confirms what was described earlier is a study carried out in humans, in which the inventor demonstrates that hypertensive individuals have changes in the levels of membrane lipids (Table 1) . The membrane lipids have an influence on the lamellar-hexagonal transition, which in its turn determines the localization and functionality of G-proteins. In fact, in patients with hypertension, we observe a change in the levels of G-proteins bound to the membrane which is due to the aforementioned change in membrane lipids and the ease of forming hexagonal phases. If the modulation of non-lamellar membrane structures and the consequent relocalization of the G-proteins produces hypertension, by regulating the lamellar-hexagonal transition of the membrane lipids it is possible to achieve regulation of the localization of the membrane proteins and, finally, of blood pressure (Fig. 3).

**Table 1.**

| **Composition of fatty acids of phospholipids and esters of cholesterol in erythrocyte membranes in normotensive (control) and hypertensive subjects (mg/100mg).** | | | | |
|---|---|---|---|---|
| Phospholipids | | | Esters | Cholesterol |
| Fatty acid | Patients with hypertension | Controls | Patients with hypertension | Controls |
| C14:0 | 0.4 ± 0.1 | 0.2 ± 0.1 | 0.9 ± 0.3 | 1.0 ± 0.2 |
| C14:1n-5 | 1.7 ± 0.2 | 2.2 ± 0.3 ** | 0.7± 0.2 | n.d.*** |
| C16:0 | 23.7 ± 0.6 | 23.1 ± 0.5 | 5.1 ± 0.6 | 14,3 ± 0.5 * |
| C16:1n-9 | 0.4 ± 0.0 | 0.3 ± 0.0 | 3.1 ± 0.6 | 2.5 ± 0.3 ** |
| C16:1n-7 | 0.5 ± 0.0 | 0.5 ± 0.1 | 2.4 ± 0.4 | 1.7 ± 0.1 *** |
| C16:4n-3 | 2.6 ± 0.3 | 2.5 ± 0.5 | n.d. | n.d. |
| | | | | |
| C18:0 | 16.6 ± 0.3 | 17.1 ± 0.6 | 3.2 ± 0.5 | 2.9 ± 0.5 |
| C18:1n-9t | 0.9 ± 0.1 | 0.6 ± 0.0*** | n.d. | n.d. |
| | | | | |
| C18: 1n-9 | 16.3 ± 0.6 | 16.0 ± 0.8 | 18.4 ± 1.2 | 16.7 ± 0.6 ** |
| C18:1n-7 | 1.8 ± 0.1 | 2.0 ± 0.2 ** | 1.5 ± 0.1 | 1.7 ± 0.2 ** |
| C18:2n-6 | 12.5 ± 0. 7 | 13.4 ± 0.7* | 45.8 ± 2.8 | 51.1 ± 1.6*** |
| C18:3n-6 | 0.4 ± 0.0 | n.d.*** | 0.9 ± 0.2 | 0.8 ± 0.1 ** |
| | | | | |
| C18:3n-3 | 0.3 ± 0.0 | 0.4 ± 0.1 | n.d. | n.d. |
| | | | | |
| C20:2n-6 | 2.1 ± 0.1 | 2.1 ± 0.2 | 0.9 ± 0.1, | 0.9 ± 0.1 |
| C20:4n-6 | 17.0 ± 0.4 | 16.5 ± 0.4 | 7.0 ± 0.6 | 6.4 ± 0.9 * |
| C22:4n-6 | 0.7 ± 0.3 | 0.6 ± 0.1 | n.d. | n.d. |
| | | | | |
| C22:6n-3 | 0.7 ± 0.1 | 0.8 ± 0.2 | n.d. | n.d. |
| | | | | |
| C24: 1n-9 | 1.5 ± 0.1 | 1.6 ± 0.1 | n.d. | n.d. |
| Total SFA | 41.2 ± 1.1 | 41.1 ± 0.7 | 19.3 ± 1.2 | 18.2 ± 1.1 * |
| Total MUFA | 22.6 ± 0.6 | 21.7 ± 0.7* | 25.9 ± 1.6 | 22.7 ± 0.8*** |
| Total PUFA | 36.2 ± 1.2 | 38.2 ± 0.9 ** | 54.8 ± 2.4 | 59.1 ± 1.6 *** |
| PUFA:SFA | 0.8 ± 0.04 | 0.9 ± 0.03 | 2.7 ± 0.3 | 3.4 ± 0.3 *** |
| PUFA: MUFA | 1.6 ±0.1 | 1.7 ± 0.1 | 2.2 ± 0.2 | 2.6 ± 0.2*** |

| | | | | |
|---|---|---|---|---|
| The values are mean values ± standard error of the mean (n=28) . SFA, saturated fatty acids; MUFA, monounsaturated fatty acids; PUFA, polyunsaturated fatty acids. *P < 0.05, **P < 0.01, ***P < 0.001. n.d.: not detected. | | | | |

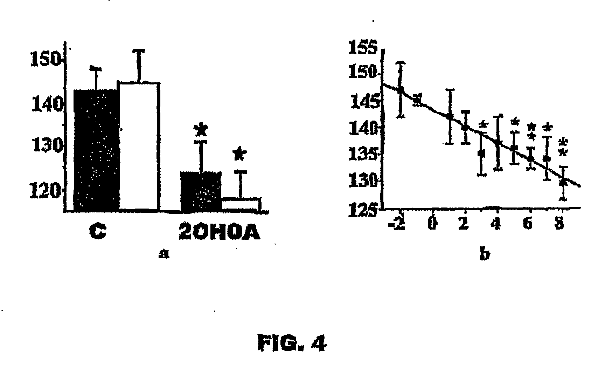

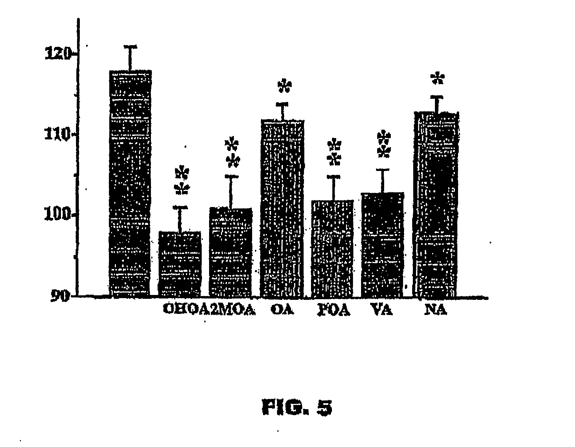

It was demonstrated that 2-hydroxyoleic acid and its analogs have a marked hypotensive effect, since they induce reductions in blood pressure, without altering the heart rate (Figs. 4a, 4b and 5). The hypotensive effect produced by ingestion of 2-hydroxyoleic acid and its analogs results in a reduction in blood pressure within 2 hours of ingestion, and this is maintained for days and weeks while ingestion of this compound is maintained. Aminoleic acid, for example, reduces blood pressure by 15 mmHg in chronic treatments lasting a week.

Blood pressure is controlled by various systems of receptors coupled to G-proteins, such as vasopressin receptors, adrenergic receptors, etc.

Interaction between the hormones involved in the control of blood pressure with the receptors bound to stimulating G-proteins is modulated by the action of fatty acids of the 2-hydroxyoleic acid type and similar functional molecules.

These fatty acids regulate communication between receptor, G-protein and effector. The result is a modulation of the signals of cyclic AMP, phospholipase C and nitric oxide, which gives rise to a reduction in blood pressure. This effect is also connected with regulation of the membrane structure. An additional advantage of these compounds is that they control other cardiovascular risk factors: the serum lipid/lipoprotein profile and body weight. Since control of blood pressure is not sufficient on its own to prolong the life of patients with hypertension, it is necessary to control other cardiovascular risk factors. Therefore, these fatty acids are unsurpassable dietary supplements for preventing the development of cardiovascular diseases.

### - Hypotensive tests -

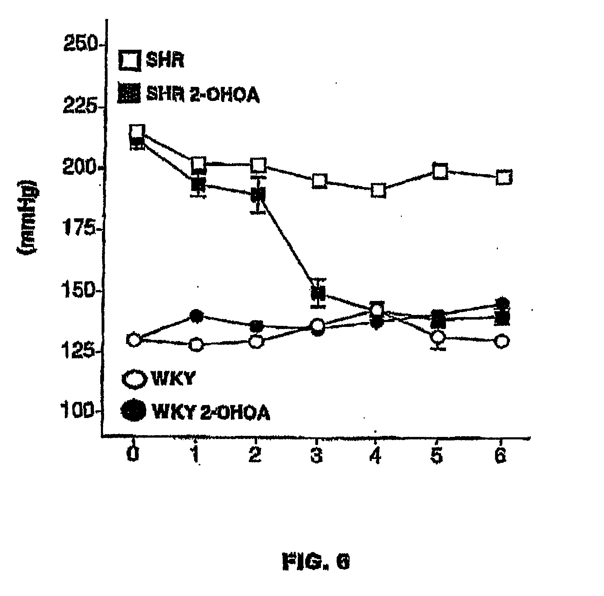

2-Hydroxyoleic acid induced significant reductions of blood pressure in rats (Fig. 4a). These reductions occurred rapidly (at 2 hours of treatment, 19±6 mmHg, P<0.01, n = 6) and after a long period (1 week, 26±7 mmHg, P<0.001, n = 6). In genetically hypertensive rats, SHR strain, ingestion of 2-hydroxyoleic acid (600 mg/kg every 12 hours) produced very marked reductions in blood pressure. These reductions, of some 70 mmHg, induced normalization of systolic pressure in hypertensive SHR rats. On the contrary, the blood pressure of all the controls used (WKY rats with and without diet and SHR rats without diet) did not change during the period of treatment (Fig. 6).

In humans, 2-hydroxyoleic acid also produced significant, large decreases in blood pressure, see Fig. 4b.

Moreover, the analogs of 2-hydroxyoleic acid that comply with the general formula given above had a hypotensive effect (Fig. 5). In all cases, the decreases in blood pressure were significant in comparison with the controls (*P<0.05, **P<0.01).

These results clearly show that 2-hydroxyoleic acid and its functional analogs can be used as food additives that improve high blood pressure.

In addition to their hypotensive action, 2-hydroxyoleic acid and its functional analogs have beneficial effects on cardiovascular parameters through the reduction of body weight (Fig. 5).

Body weight is regulated by, among other things, factors such as the individual's metabolic capacity and control of food intake.

Control of food intake is determined by the feeling of satiety, which in turn is regulated at the hormonal level. For example, deficiency of nutrients stimulates the secretion of hormones which give rise to a sensation of appetite. After eating, once the nutrient levels have been restored, there is stimulation of the secretion of hormones that give rise to a feeling of satiety.

It has been found that fatty acids of the 2-hydroxyoleic acid type and their functional analogs produce effects of satiety, inducing reductions in food intake. This control is also mediated by receptors of cytokines, leptins, adrenoceptors, and other receptors coupled to G-proteins, whose activity is modulated by these fatty acids. These results are in line with the effects of the Mediterranean diet (rich in monounsaturated fatty acids such as hydroxyoleic acid and derivatives) on body weight. In this regard, it is known that obesity is less widespread in Mediterranean countries than in Northern countries.

Owing to the control exerted by hydroxyoleic acid and derivatives on ingestion, the animals receiving nutritional supplements of hydroxyoleic acid and derivatives lost weight, despite having free access to food and water. This effect on satiety meant a consumption of feed between 15% and 30% less than the control animals.

### - Tests of control of body weight -

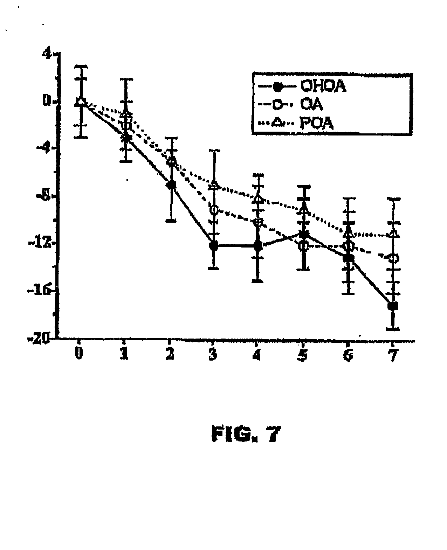

Rats receiving a nutritional supplement containing the molecules of the invention lost body weight during food-intake periods of from 5 to 17 days. In these experiments, rats were given 2-hydroxyoleic acid or its functional analogs, in particular aminoleic acid, and had free access to food and water, in the same way as the control group of rats, which received the same diet without the addition of fatty acids (Fig. 7). In these conditions, the body weight of rats receiving the supplement progressively decreased, starting from the first day, up to 17 grams on the seventh day (5% of the normal body weight of a Sprague-Dawley rat aged 2-3 months) . The feed supplied to these animals was weighed and the consumption was found to be lower during the treatment time, confirming that the treatments with the molecules relating to this invention produce an effect of satiety in the animal. Similar experiments carried out on adult mice, for periods of up to 28 days with 2-hydroxyoleic acid, show reductions in body weight from 15% to 25%, relative to control mice (without nutritional supplement).

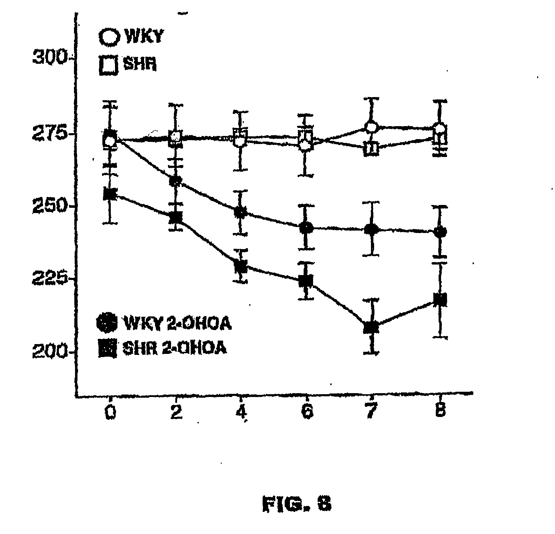

In another series of experiments, the diet of normotensive rats (WKY) and hypertensive rats (SHR) was supplemented with hydroxyoleic acid (600 mg/kg, every 12 hours) and it was confirmed that the weight of the animals decreased by between 30 and 40 grams (15-20% of the body weight) in an 8-day diet period (Fig. 8).

### Description of the figures

Fig. 1: Some of the many structures, in addition to lamellar, that the membranes can adopt. A) Forms of availability of lipid molecules; B) Molecular organizations: (L) lamellar with predominance of phosphatidylcholine; (H_{I}) (H_{II}) hexagonal with predominance, respectively, of lysophosphatidylcholine and of phosphatidylethanolamine.
Fig. 2 shows the binding of [³⁵S] GTPγS to membranes of NIH 3T3 cells transfected with the rat adrenoceptor α_{2A/D}. This parameter measures the activity of G-proteins. The presence of 2-hydroxyoleic acid (2OHOA) induces a decrease in function of the G-proteins even greater than daunomycin (DNM).
   Abscissa: C = control; 2OHOA = 2-hydroxyoleic; DNM = daunomycin.
   Ordinate: Binding of [³⁵S]GTPγS (% of control).
Fig. 3: Levels of G-proteins in membranes of erythrocytes, measured as % of control, of normotensive subjects (empty bars) and hypertensive subjects (filled bars). The levels of proteins Gαi_{1/2}(Gi), Gαo (Go), Gαs (Gs) and G Gβ (Gb) are significantly lower in hypertensive subjects. The values of the bars are mean values ± standard error of the mean *P<0.05, **P<0.01.
Fig. 4a shows the acute effect (30 mg/kg, 2 hours, black bars) and chronic effect (90 mg/kg per day, for 7 days, white bars) of hydroxyoleic acid (20HOA) on the systolic arterial pressure (mmHg) in female Sprague-Dawley rats. * P<0.01, with respect to the control (C).
Fig. 4b shows the effect of 2-hydroxyoleic acid (90 mg/kg per day, for 8 days) on blood pressure (mmHg) in humans. This diagram shows systolic arterial pressure as a function of the day of treatment. The days prior to treatment are shown with negative values. *P<0.05, **P<0.01.
Fig. 5 shows the effect of the short-term (2 to 4 hours) administration of 2-hydroxyoleic acid (20HOA) and its functional analogs 2-methyl oleic acid (2MOA), oleic acid (OA), palmitoleic acid (POA), cis-vaccenic acid (VA) and nervonic acid (NA). All the treatments carried out with 2-hydroxyoleic acid and the analogs mentioned induced significant decreases (*P<0.05, **P<0.001) in systolic arterial pressure (mmHg) in female Sprague-Dawley rats, with respect to the control (C).
Figure 6 shows the effect of 2-hydroxyoleic acid on systolic arterial pressure (mmHg) in male genetically hypertensive rats of the strain SHR; (SHR 2OHOA, black squares). The consumption of 2-hydroxyoleic acid (600 mg/kg, every 12 hours for 7 days) induced significant, large reductions in systolic arterial pressure. In SHRs which were not given 2-hydroxyoleic acid (SHR empty squares), no reduction in arterial pressure was observed. In males of the WKY strain (normotensive), neither the 2-hydroxyoleic acid (black circles) nor the vehicle (empty circles) produced statistically significant changes in arterial pressure. The reduction induced by hydroxyoleic acid on arterial pressure meant that there was no significant difference between the values registered at the end of the diet period for the hypertensive and normotensive rats.
Figure 7 shows the effect of 2-hydroxyoleic acid (20HOA) and its analogs, oleic acid (OA) and palmitoleic acid (POA) (90 mg/kg daily), on the body weight measured in decrease in grams per day of treatment. The animals (Sprague-Dawley rats) had free access to food and water at all times.
Figure 8 shows the effect of hydroxyoleic acid (black circles and squares) (600 mg/kg, every 12 hours for 7 days) on the body weight of hypertensive (SHR) and normotensive (WKY) rats. After eight days of hydroxyoleic intake, both strains showed considerable reductions in body weight (15-20% reduction), whereas the control animals (white circles and squares) treated with the vehicle (without 20HOA) did not undergo any significant changes in body weight.

## Claims

1. Use of compounds of general formula I: COOH-CHR-(CH₂)ₘ-CH=CH-(CH₂)ₙ-CH₃, where R is OH and m and n have, independently, a value between 0 and 15, in the manufacture of food additives and/or ingredients, dietary products, acceptable food forms, functional food additives and foods in general, the ingestion of which is useful for the prevention and control of hypertension and/or obesity.

2. Use according to Claim 1, **characterized in that** the compounds of general formula I preferably have between 12 and 28 carbon atoms.

3. Use according to any one of Claims 1 or 2 **characterized in that** the compound of general formula I is preferably 2-hydroxyoleic acid.

4. Use according to claim 3, in which the ingestion of 2-hydroxyoleic acid, as food additive and/or ingredient dietary product, acceptable food form, functional food additive or food in general, is useful in the prevention and control of hypertension.

5. Use according to claim 3, in which the ingestion of 2-hydroxyoleic acid, as food additive and/or ingredient dietary product, acceptable food form, functional food additive or food in general, is useful in the prevention and control of obesity.

## Patentansprüche

1. Verwendung von Verbindungen der allgemeinen Formel 1: COOH-CHR-(CH₂)ₘ-CH=CH-(CH₂)ₙ-CH₃, wobei R OH ist und m und n unabhängig voneinander einen Wert zwischen 0 und 15 haben, in der Herstellung von Nahrungsergänzungsmitteln und/oder Nahrungsinhaltsstoffen, Diätprodukten, verträglichen Nahrungsformen, funktionellen Nahrungsergänzungsmitteln oder Nahrungsmitteln im Allgemeinen, deren Aufnahme zur Vorbeugung und Kontrolle von Bluthochdruck und/oder Fettleibigkeit von Nutzen ist,

2. Verwendung nach Anspruch 1, **dadurch gekennzeichnet, dass** die Verbindungen der allgemeinen Formel I vorzugsweise zwischen 12 und 28 Kohlenstoffatome haben.

3. Verwendung nach einem der Ansprüche 1 oder 2, **dadurch gekennzeichnet, dass** die Verbindungen der allgemeinen Formel I vorzugsweise 2-Hydroxyölsäure ist.

4. Verwendung nach Anspruch 3, wobei die Aufnahme von 2-Hydroxyölsäure als Nahrungsergänzungsmittel und/oder Nahrungsinhaltsstoff, Diätprodukt, verträgliche Nahrungsform, funktionelles Nahrungsergänzungsmittel oder Nahrungsmittel im Allgemeinen bei der Vorbeugung und Kontrolle von Bluthochdruck von Nutzen ist.

5. Verwendung nach Anspruch 3, wobei die Aufnahme von 2-Hydroxyölsäure als Nahrungsergänzungsmittel und/oder Nahrungsinhaltsstoff, Diätprodukt, verträgliche Nahrungsform, funktionelles Nahrungsergänzungsmittel oder Nahrungsmittel im Allgemeinen bei der Vorbeugung und Kontrolle von Fettleibigkeit von Nutzen ist.

## Revendications

1. Utilisation de composés de formule générale I : COOH-CHR-(CH₂)ₘ-CH=CH-(CH₂)ₙ-CH₃, dans laquelle R représente un groupe OH et m et n ont, indépendamment, une valeur de 0 à 15, dans la production d'additifs et/ou d'ingrédients alimentaires, de produits diététiques, de formes d'aliments acceptables, d'additifs alimentaires fonctionnels et d'aliments en général, dont l'ingestion est utile pour la prévention de, et la lutte contre, l'hypertension et/ou l'obésité.

2. Utilisation suivant la revendication 1, **caractérisée en ce que** les composés de formule générale I ont de préférence 12 à 28 atomes de carbone.

3. Utilisation suivant l'une quelconque des revendications 1 et 2, **caractérisée en ce que** le composé de formule générale I est de préférence l'acide 2-hydroxyoléique.

4. Utilisation suivant la revendication 3, dans laquelle l'ingestion d'acide 2-hydroxyoléique, comme additif et/ou ingrédient alimentaire, produit diététique, forme d'aliment acceptable, additif alimentaire fonctionnel ou aliment en général, est utile dans la prévention de, et la lutte contre, l'hypertension.

5. Utilisation suivant la revendication 3, dans laquelle l'ingestion d'acide 2-hydroxyoléique, comme additif et/ou ingrédient alimentaire, produit diététique, forme d'aliment acceptable, additif alimentaire fonctionnel ou aliment en général, est utile dans la prévention de, et la lutte contre, l'obésité.
